(19) Europäisches Patentamt
European Patent Office
Office européen des brevets

(11) Veröffentlichungsnummer: **0 016 949**
**B1**

(12) **EUROPÄISCHE PATENTSCHRIFT**

(45) Veröffentlichungstag der Patentschrift: **14.10.81**

(51) Int. Cl.³: **C 07 C 125/065**

(21) Anmeldenummer: **80100849.1**

(22) Anmeldetag: **21.02.80**

(54) **Verfahren zur Herstellung von Urethanen.**

(30) Priorität: **02.03.79 DE 2908252**

(43) Veröffentlichungstag der Anmeldung: **15.10.80 Patentblatt 80/21**

(45) Bekanntmachung des Hinweises auf die Patenterteilung: **14.10.81 Patentblatt 81/41**

(84) Benannte Vertragsstaaten: **AT BE CH DE FR GB IT NL**

(56) Entgegenhaltungen: **US-A-2 409 712**

(73) Patentinhaber: **BAYER AG, Zentralbereich Patente, Marken und Lizenzen, D-5090 Leverkusen 1, Bayerwerk (DE)**

(72) Erfinder: **Becker, Robert, Dr., Fichtestrasse 2a, D-5090 Leverkusen (DE)**
Erfinder: **Grolig, Johann, Dr., Heinrich-Lübke-Strasse 22, D-5090 Leverkusen (DE)**
Erfinder: **Rasp, Christian, Dr., Wolfskaul 10, D-5000 Koeln 80 (DE)**
Erfinder: **Stammann, Günter, Dr., Silesiusstrasse 78, D-5000 Koeln 80 (DE)**

**EP 0 016 949 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von Urethanen

Die vorliegende Erfindung betrifft ein verbessertes Verfahren zur Herstellung von Urethanen au N,N'-disubstituierten Harnstoffen und mindestens eine Hydroxylgruppe enthaltenden organische Verbindungen.

Organische Isocyanate werden großtechnisch im allgemeinen durch Umsetzung der entsprechen den Amine mit Phosgen hergestellt. Wegen der Giftigkeit des Phosgens ist man seit geraumer Ze bestrebt, einen großtechnisch gangbaren Syntheseweg zu organischen Isocyanaten aufzufinden, be welchem sich die Verwendung des Phosgens erübrigt. Ein derartiger Syntheseweg besteht in de Umsetzung von organischen Nitroverbindungen mit Kohlenmonoxid und organischen Hydroxylverbin dungen zu den entsprechenden Urethanen und deren anschließende Spaltung in Isocyanat un Hydroxylgruppen aufweisende Verbindungen, wobei vor der Spaltung auch eine Modifizierung des al Zwischenprodukt erhaltenen Urethans denkbar ist. So ist es beispielsweise möglich, das au Nitrobenzol, Kohlenmonoxid und Äthanol zugängliche Phenylurethan zunächst mit Formaldehyd zur Bisurethan des 4,4'-Diisocyanatodiphenylmethans umzusetzen, um das so erhaltene Zwischenproduk unter Abspaltung des Äthanols in 4,4'-Diisocyanatodiphenylmethan zu überführen.

Die Spaltung von Urethanen in die entsprechenden Isocyanate und Hydroxylgruppen aufweisende Verbindungen ist beispielsweise in der DE-OS 2 421 503 bzw. in den in dieser Literaturstell abgehandelten Vorveröffentlichungen beschrieben.

Für die Herstellung der Urethane sind in der Patentliteratur die Umsetzung von Nitroverbindunge mit Kohlenmonoxid und Alkoholen in Gegenwart von Selen oder Selenverbindungen (DE-OS 2 343 826 2 614 101, 2 623 694) oder von Edelmetallen, insbesondere Palladium, in Gegenwart von Lewissäure (DE-OS 1 568 044 und 2 603 574) beschrieben. Diese Umsetzung verläuft im Falle der Verwendung eine Mononitroverbindung nach folgender Stöchiometrie:

$$R-NO_2+3\,CO+R'OH \rightarrow RNHCO_2R'+2\,CO_2$$

Die allgemeine Reaktionsgleichung lautet:

$$R(NO_2)_x+3\,xCO+xR'OH \rightarrow R(NHCO_2R')_x+2\,xCO_2.$$

Das heißt, daß pro Mol an zu erzeugender Urethangruppe 3 Mole Kohlenmonoxid verbraucht und Mole Kohlendioxid gebildet werden. Das eingesetzte Kohlenmonoxid wird somit nur zu einem Dritte für die Herstellung der Urethangruppierung genutzt, während zwei Drittel in das technisch wertlos inerte Kohlendioxid umgewandelt werden. Bedingt durch die hohe Wärmetönung der exotherme Bildung von $CO_2$ sind bei der technischen Durchführung der bekannten Urethan-Synthese auf de Basis von Nitroverbindung, Kohlenmonoxid und Alkohol aufwendige Vorrichtungen zur Abführung de hohen Reaktionswärme nötig.

Aus der Patentliteratur (US-Patent 2 409 712) ist weiterhin bekannt, N,N'-Dialkylharnstoffe ode N,N'-Diarylharnstoffe mit Alkoholen zu einem Urethan und einem Amin umzusetzen:

$$RNHCONHR+R'OH \rightarrow RNHCO_2R'+RNH_2$$

Bei dieser Arbeitsweise wird jedoch nur die Hälfte der im Harnstoff enthaltenen Alkyl- ode Arylgruppen ins Urethan überführt, während die andere Hälfte zum freien Amin umgesetzt wird.

Es wurde nun gefunden, daß N,N'-disubstituierte Harnstoffe mit mindestens eine Hydroxylgrupp enthaltenden organischen Verbindungen ohne Abspaltung von freiem Amin, d. h. praktisch unte Verdoppelung der Ausbeute bezogen auf N,N'-disubstituierten Harnstoff in Urethane überführ werden können, wenn die Harnstoffe zusammen mit der mindestens eine Hydroxylgrupp enthaltenden organischen Verbindung mit Kohlenmonoxid unter bestimmten, nachstehend nähe erläuterten Reaktionsbedingungen einer Oxicarbonylierung unterzogen werden.

Gegenstand der vorliegenden Erfindung ist ein Verfahren zur Herstellung von Urethanen durc Umsetzung von N,N'-disubstituierten Harnstoffen und mindestens eine Hydroxylgruppe enthaltende organischen Verbindungen, dadurch gekennzeichnet, daß man diese Ausgangsverbindungen mi Kohlenmonoxid einer Oxicarbonylierung in Gegenwart von

a) molekularem Sauerstoff und/oder organischen Nitroverbindungen als Oxidationsmittel und
b) eines Katalysatorsystems, welches
ba) aus einem Edelmetall und/oder einer Edelmetallverbindung der 8. Nebengruppe de Periodensystems der Elemente und
bb) einer unter den Reaktionsbedingungen zu Redoxreaktionen befähigten, von den Verbindunge ba) verschiedenen Verbindung von Elementen der 3. bis 5. Hauptgruppe und/oder der 1. bi 8. Nebengruppe des Periodensystems der Elemente besteht,

unterwirft.

Ausgangsmaterialien für das erfindungsgemäße Verfahren sind beliebige mit einem organischen Rest substituierte N,N'-disubstituierte Harnstoffe, die gegebenenfalls noch Nitrogruppen enthalten können, wodurch ein weiterer Zusatz von Nitroverbindungen entfallen kann, eines im allgemeinen zwischen 88 und 500, vorzugsweise 212 bis 330, liegenden Molekulargewichts, sowie beliebige mindestens eine Hydroxygruppe enthaltende organische Verbindungen, beispielsweise substituierte, aliphatische, cycloaliphatische und/oder aromatische Mono- oder Polyhydroxyverbindungen eines im allgemeinen zwischen 32 und 300, vorzugsweise 32 und 102, liegenden Molekulargewichts.

Die organischen Substituenten der N,N'-disubstituierten Harnstoffe können gleich oder verschieden sein; es kann sich um aliphatische, cycloaliphatische oder armoatische Reste handeln, wie beispielsweise Methyl, Ethyl, Ethylen, Propyl, Isopropyl, 1,2-Propylen, 1,3-Propylen, Butyl, Isobutyl, sec.-Butyl, tert.-Butyl, Pentyl, Hexyl, Heptyl, Octyl, Decyl, Dodecyl, Cetyl, Cyclopentyl, Cyclohexyl, Phenyl, p-Tolyl, o-Tolyl, m-Tolyl, o-Chlorphenyl, p-Chlorphenyl, m-Chlorphenyl, o-Aminophenyl, p-Aminophenyl, m-Aminophenyl, o-Nitrophenyl, p-Nitrophenyl, m-Nitrophenyl, 3-Amino-2-methylphenyl, 3-Amino-4-methylphenyl, 5-Amino-2-methylphenyl, 3-Nitro-2-methylphenyl, 3-Nitro-4-methylphenyl, 5-Nitro-2-methylphenyl, 1-Naphthyl, 2-Naphthyl, 6-Amino-1-naphthyl, 6-Nitro-1-naphthyl u. ä.

Bevorzugt sind N,N'-disubstituierte Harnstoffe mit aromatischen Resten, wie beispielsweise folgende N,N'-Diarylharnstoffe: N,N'-Diphenylharnstoff, N,N'-Di-(p-tolyl)-harnstoff, N,N'-Di-(o-tolyl)-harnstoff, N,N'-Di-(o-aminophenyl)-harnstoff, N,N'-Di-(p-aminophenyl)-harnstoff, N,N'-Di-(o-nitrophenyl)-harnstoff, N,N'-Di-(p-nitrophenyl)-harnstoff, N,N'-Di-(3-amino-2-methylphenyl)-harnstoff, N,N'-Di-(3-amino-4-methylphenyl)-harnstoff, N,N'-Di-(5-amino-2-metnylphenyl)-harnstoff, N,N'-Di-(3-nitro-4-methylphenyl)-harnstoff, N,N'-Di-(5-nitro-2-methylphenyl)-harnstoff, N,N'-Di-(3-nitro-2-methylphenyl)-harnstoff, N-(3-Amino-2-methylphenyl-N'-(3-nitro-2-methylphenyl)-harnstoff, N-(3-Amino-4-methylphenyl)-N'-(3-nitro-4-methylphenyl)-harnstoff, N-(5-Amino-2-methylphenyl-N'-(5-nitro-2-methylphenyl)-harnstoff, N-(3-Amino-2-methylphenyl)-N'-(5-amino-2-methylphenyl)-harnstoff, N-(3-Amino-2-methylphenyl)-N'-(3-amino-4-methylphenyl)-harnstoff, N-(3-Amino-4-methylphenyl)-N'-(5-amino-2-methylphenyl)-harnstoff, N-(3-Nitro-2-methylphenyl)-N'-(5-amino)-2-methylphenyl)-harnstoff, N-(3-Nitro-2-methylphenyl)-N'-(3-nitro-4-methylphenyl)-harnstoff oder N-(3-Nitro-4-methylphenyl)-N'-(5-Nitro-2-methylphenyl)-harnstoff. Besonders bevorzugt sind N,N'-Diphenylharnstoff, N,N'-Di-(p-tolyl)-harnstoff, N,N'-Di-(o-tolyl)-harnstoff, N,N'-Di-(3-amino-2-methylphenyl)-harnstoff, N,N'-Di-(3-amino-4-methylphenyl)-harnstoff und N,N'-Di-(5-amino-2-methylphenyl)-harnstoff, oder auch die entsprechenden unsymmetrischen N,N'-disubstituierten an den aromatischen Ringen amino- und methylsubstituierten Diarylharnstoffe bzw. beliebige Gemische dieser Verbindungen.

Ausgangsmaterialien für das erfindungsgemäße Verfahren sind des weiteren beliebige Hydroxylgruppen aufweisende organische Verbindungen wie z. B. beliebige ein- oder mehrwertige Alkohole bzw. ein- oder mehrwertige Phenole. Geeignete Alkohole sind beispielsweise solche des Molekulargewichtsbereichs 32 via 300. Es kann sich bei diesen Alkoholen um beliebige lineare oder verzweigte ein- oder mehrwertige Alkanole oder Alkenole, um beliebige ein- oder mehrwertige Cycloalkanole, Cycloalkenole oder Aralkylalkohole handeln.

Auch beliebige inerte Substituenten aufweisende Alkohole sind geeignet. Geeignete Substituieten sind z. B. Halogenatome, Sulfoxidgruppen, Sulfongruppen, Carbonyl- oder Carbonsäureestergruppen. Auch Etherbrücken aufweisende Alkohole sind grundsätzlich geeignet. Beispiele geeigneter Alkohole sind: Methanol, Ethanol, n-Propanol, Isopropanol, n-Butanol, n-Pentanol, n-Hexanol, Cyclohexanol, Benzylalkohol, Chlorethanol, Ethylenglykol, Diethylenglykol, Propylenglykol, Dipropylenglykol, Glycerin, Hexantriol oder Trimethylolpropan. Bevorzugt werden einwertige aliphatische Alkohole mit 1 bis 6 Kohlenstoffatomen eingesetzt.

Geeignete Phenole sind insbesondere solche eines Molekulargewichtsbereichs 94—300 wie beispielsweise Phenol, die isomeren Chlorphenole, Kresole, Ethylphenole, Propylphenole, Butylphenole oder höheren Alkylphenole, Brenzcatechin, 4,4'-Dihydroxydiphenylmethan, Bisphenol-A, Anthranol, Phenanthrol, Pyrogallol oder Phloroglucin. Die beispielhaft genannten Alkohole sind gegenüber den beispielhaft genannten Phenolen bevorzugt. Ethanol ist die besonders bevorzugte beim erfindungsgemäßen Verfahren einzusetzende Hydroxylgruppen aufweisende Verbindung.

Die organischen Hydroxyverbindungen werden bei der Durchführung des erfindungsgemäßen Verfahrens im allgemeinen in solchen Mengen eingesetzt, daß für jede zu bildende Urethangruppe 1 bis 100 Hydroxylgruppen im Reaktionsgemisch vorliegen. Hierbei ist zu berücksichtigen, daß bei Verwendung von Amino-substituierten Harnstoffen bzw. bei Vorliegen von Nitroverbindungen im Reaktionsgemisch die Amino- und Nitrogruppen ebenfalls in Urethangruppen überführt werden, so daß für jedes Mol Harnstoff, sowie für jedes Mol Amino- oder Nitrogruppen jeweils ein Äquivalent der Hydroxylgruppe bei stöchiometrischem Umsatz benötigt wird.

Als weiterer Reaktionspartner kommt beim erfindungsgemäßen Verfahren Kohlenmonoxid zum Einsatz. Dieses Ausgangsmaterial wird im allgemeinen in einer Menge eingesetzt, die 1 bis 30 Mol Kohlenmonoxid pro Mol zu erzeugendes Urethan entspricht. Hierbei ist zu berücksichtigen, daß bei stöchiometrischer Reaktion für jedes im Reaktionsgemisch vorliegendes Mol Harnstoff und jedes Mol an Amino- oder Nitrogruppen jeweils 1 Mol Kohlenmonoxid benötigt werden.

Die erfindungsgemäße Umsetzung erfolgt in Gegenwart von a) Oxidationsmitteln und b)

Katalysatoren.

Als Oxidationsmittel kann molekularer Sauerstoff in reiner Form oder in Form von Gemischen mit Inertgasen, wie Stickstoff oder Kohlendioxid, insbesondere in Form von Luft, eingesetzt werden. In Gegenwart von molekularem Sauerstoff erfolgt Oxicarbonylierung beispielsweise nach folgender Reaktionsgleichung ($R^1$ verschieden von $R^2$ oder bevorzugt $R^1 = R^2$):

$$R^1NHCONHR^2 + \frac{1}{2} O_2 + CO + 2 R'OH \longrightarrow R^1NHCO_2R' + R^2NHCO_2R' + H_2O$$

Bevorzugte Oxidationsmittel sind organische Nitroverbindungen. Man kann die verschiedensten Nitroverbindungen verwenden, wobei diese unter den Bedingungen der Oxicarbonylierung ebenfalls zu Urethanen umgesetzt werden, beispielsweise nach folgender Reaktionsgleichung:

$$2 R^1NHCONHR^2 + R^3NO_2 + 3 CO + 5 R'OH \longrightarrow 2 R^1NHCO_2R' + 2 R^2NHCO_2R' + R^3NHCO_2R' + 2 H_2O$$

Ganz besonders bevorzugt wird die Umsetzung von symmetrischen N,N'-Diarylharnstoffen in Gegenwart einer aromatischen Nitroverbindung durchgeführt, deren Arylrest den Arylresten des Harnstoffs entspricht. Dabei erfolgt die Oxicarbonylierung nach folgender Gleichung unter Bildung eines einzigen Urethans:

$$2 RNHCONHR + RNO_2 + 3 CO + 5 R'OH \longrightarrow 5 RNHCO_2R' + 2 H_2O$$

Im Interesse einer möglichst optimalen Ausbeute werden bei Verwendung von organischen Nitroverbindungen als alleinigem Oxidationsmittel die Mengen der Harnstoffverbindung und der Nitroverbindung vorzugsweise so bemessen, daß im Reaktionsgemisch auf jedes Mol an Nitrogruppen 2 Mol Harnstoff entfallen. Beim Einsatz von Amin-substituierten Harnstoffen ist jedoch darauf zu achten, daß zur Überführung der Aminogruppen in Urethangruppen für jedes Mol an Aminogruppen zusätzlich ein halbes Mol an Nitrogruppen erforderlich sind. Selbstverständlich kann bei Verwendung von Nitro-substituierten Harnstoffen auf den Zusatz eines weiteren Oxidationsmittels verzichtet werden, falls die vorhandenen Nitrogruppen stöchiometrisch für die Umsetzung zum Urethan ausreichen.

Bei Verwendung von molekularem Sauerstoff als alleinigem Oxidationsmittel ist im Interesse einer möglichst optimalen Ausbeute dafür zu sorgen, daß entsprechend obiger Reaktionsgleichung für jedes Mol Harnstoff mindestens ein halbes Mol Sauerstoff und bei Vorliegen von Aminogruppen für jedes Mol Aminogruppen ebenfalls mindestens ein halbes Mol Sauerstoff zur Verfügung steht. Überschüssige Mengen an Sauerstoff sind möglich, unterschüssige Mengen an Sauerstoff würden zu Ausbeuteverlusten führen. Vorzugsweise wird ein Inertgas wie Stickstoff oder Kohlendioxid zugegeben, wobei die Mengen so bemessen werden, daß die Reaktion außerhalb des Explosionsbereichs der Sauerstoff-Kohlenoxid-Gemische, bzw. der Sauerstoff-Alkohol-Gemische durchgeführt werden kann. Ohne Inertgaszugabe ist die Sauerstoffzugabe so zu bemessen, daß explosible Gemische mit dem Kohlenmonoxid und der Alkohol-Komponente vermieden werden. Bevorzugt wird der molekulare Sauerstoff in Form von Luft oder Luft-Stickstoff-Gemischen eingesetzt.

Selbstverständlich können auch Sauerstoff und Nitroverbindung gleichzeitig als Oxidationsmittel eingesetzt werden. In einem solchen Falle ist selbstverständlich auch die Verwendung von Nitroverbindungen in geringeren als den stöchiometrischen Mengen möglich. Die Oxidationsmittel können selbstverständlich auch im Überschuß zugegeben werden, so daß bei Verwendung von Nitroverbindungen ganz allgemein gesagt werden kann, daß die Nitroverbindungen im allgemeinen in solchen Mengen zum Einsatz gelangen, daß das Molverhältnis zwischen Harnstoff und Nitrogruppen zwischen 1 : 1 und 4 : 1, insbesondere zwischen 1,5 : 1 und 2,5 : 1 und ganz besonders bevorzugt zwischen 1,8 : 1 und 2,2 : 1 liegt.

Für das erfindungsgemäße Verfahren geeignete Nitroverbindungen sind beliebige Nitrogruppen aufweisende, organische Verbindungen mit mindestens einer aliphatisch, cycloaliphatisch und/oder aromatisch gebundenen Nitrogruppe, eines im allgemeinen zwischen 61 und 400, vorzugsweise 123 und 262 liegenden Molekulargewichts.

Beispielsweise können folgende aromatische Nitroverbindungen eingesetzt werden: Nitrobenzol, o-Dinitrobenzol, m-Dinitrobenzol, p-Dinitrobenzol, o-Chlor-nitrobenzol, m-Chlor-nitrobenzol, o-Chlornitrobenzol, o-Nitrotoluol, m-Nitrotoluol, p-Nitrotoluol, o-Amino-nitrobenzol, m-Amino-nitrobenzol, p-Amino-nitrobenzol, 2-Amino-3-nitrotoluol, 3-Amino-2-nitrotoluol, 2-Amino-4-nitrotoluol, 4-Amino-2-nitrotoluol, 2-Amino-5-nitrotoluol, 2-Amino-6-nitrotoluol, 5-Amino-2-nitrotoluol, 3-Amino-4-nitrotoluol, 4-Amino-3-nitrotoluol, 3-Amino-4-nitrotoluol, 3-Amino-5-nitrotoluol, 2,3-Dinitrotoluol, 2,4-Dinitrotoluol, 2,5-Dinitrotoluol, 2,6-Dinitrotoluol, 3,4-Dinitrotoluol, 3-Nitro-o-xylol, 4-Nitro-o-xylol, 2-Nitro-m-xylol, 4-Nitro-m-xylol, 5-Nitro-m-xylol, Nitro-p-xylol, 3,4-Dinitro-o-xylol, 3,5-Dinitro-o-xylol, 3,6-Dinitro-o-xylol, 4,5-Dinitro-o-xylol, 2,4-Dinitro-m-xylol, 2,5-Dinitro-m-xylol, 4,5-Dinitro-m-xylol, 4,6-Dinitro-m-xylol,

2,3-Dinitro-p-xylol, 2,6-Dinitro-p-xylol, 1-Nitronaphthalin, 2-Nitronaphthalin, Dinitronaphthaline, Nitroanthracene, Nitrodiphenyle, Bis-(nitrophenyl)-methane, Bis-(nitrophenyl)-thioäther, Bis-(nitrophenyl)-sulfone, Nitrodiphenoxyalkane, Nitrophenothiazine.

Als cycloaliphatische Nitroverbindungen seien genannt: Nitrocyclobutan, Nitrocyclopentan, Nitrocyclohexan, 1,2-Dinitrocyclohexan, 1,3-Dinitrocyclohexan, 1,4-Dinitrocyclohexan, Bis-(nitrocyclohexyl)-methane.

Beispielhaft für die Gruppe der Nitroalkane seien genannt: Nitromethan, Nitroäthan, 1-Nitropropan, 2-Nitropropan, Nitrobutane, Nitropentane, Nitrohexane, Nitrodecane, Nitrocetane, 1,2-Dinitroäthan, 1,2-Dinitropropan, 1,3-Dinitropropan, Dinitrobutane, Dinitropentane, Dinitrohexane, Dinitrodecane, Phenylnitromethan, Bis-(nitromethyl)-cyclohexane, Bis-(nitromethyl)-benzole, $\omega$-Nitrocarbonsäurenitrile.

Bevorzugte Nitroverbindungen für das erfindungsgemäße Verfahren sind aromatische Nitroverbindungen der oben beispielhaft genannten Art. Besonders bevorzugte Nitroverbindungen sind Nitrobenzol, 1,3-Dinitrobenzol, 2,4-Dinitrotoluol, 2,6-Dinitrotoluol oder z. B. 1,5-Dinitronaphthalin.

Die beim erfindungsgemäßen Verfahren zum Einsatz gelangenden Katalysatorensysteme b) enthalten als wesentlichen Bestandteil ba) Edelmetalle der achten Nebengruppe des Periodensystems und bb) eine Cokatalysator-Komponente.

Bei der Katalysatorenkomponente ba) handelt es sich entweder um freie Edelmetalle der achten Nebengruppe des Periodensystems oder um im Reaktionsgemisch lösliche Verbindungen dieser Metalle. Besonders vorteilhaft werden die Edelmetalle als im Reaktionsgemisch lösliche Verbindungen zugegeben, beispielsweise als Chloride, Bromide, Jodide, Chlorokomplexe, Bromokomplexe, Jodokomplexe, Acetate, Acetylacetonate u. a. lösliche Edelmetallverbindungen. Geeignete Edelmetalle sind Ru, Rh, Pd, Os, Ir und Pt. Bevorzugte Edelmetalle sind Palladium und Rhodium. Besonders bevorzugt wird Palladium, insbesondere in Form von löslichem Palladiumchlorid, eingesetzt. Die bevorzugten Konzentrationen, bezogen auf das Reaktionsgemisch inklusive gegebenenfalls mitverwendetem Lösungsmittel liegen im allgemeinen bei 0,0001 bis 0,1 Gew.-%, insbesondere 0,001 bis 0,01 Gew.-%, berechnet als Edelmetall. Bei niedrigeren Edelmetallkonzentrationen wird die Reaktionsgeschwindigkeit zu gering. Höhere Edelmetall-Konzentrationen sind zwar möglich, jedoch wegen der möglichen Edelmetallverluste unwirtschaftlich, da ohnehin eine weitere Steigerung der Urethanausbeuten nicht mehr erfolgt.

Bei den Cokatalysatoren bb) handelt es sich um beliebige unter den Reaktionsbedingungen zu Redoxreaktionen befähigte, von den Verbindungen ba) verschiedene Verbindungen von unter den Reaktionsbedingungen zu Redoxreaktionen befähigten Elementen der 3. bis 5. Haupt- und 1. bis 8. Nebengruppe des Periodensystems. Die entsprechenden Verbindungen stellen vorzugsweise Chloride, Oxichloride, Oxide und/oder Hydroxide der genannten Elemente dar, wobei im Falle des Einsatzes der entsprechenden Oxide bzw. Hydroxide vorzugsweise bestimmte aktivierend wirkende Chloride mitverwendet werden.

Geeignete Cokatalysatoren sind beispielsweise Kupfer(II)-chlorid, Thallium(III)-chlorid, Zinn(II)-chlorid, Zinn(IV)-chlorid, Arsen(III)-chlorid, Wismut(III)-chlorid, Vanadin(III)-chlorid, Chrom(III)-chlorid, Molybdän(IV)-chlorid, Wolfram(V)-chlorid, Wolfram(VI)-chlorid, Mangan(II)-chlorid, Eisen(II)-chlorid, Eisen(III)-chlorid, Eisenoxichlorid, Kobalt(II)-chlorid, Kupfer(II)-oxid, Kupfer(II)-hydroxid, Thallium(I)-hydroxid, Zinn(II)-oxid, Zinn(II)-hydroxid, Vanadinpentoxid, Molybdäntrioxid, Wolframtrioxid, Mangandioxid, Eisen(II)-oxid, Eisen(II)-hydroxid, Eisen(III)-hydroxid, Eisen(III)-oxide, wie z. B. $\alpha$-$Fe_2O_3$, $\gamma$-$Fe_2O_3$, Eisen(III)-oxidhydrate, wie z. B. $\alpha$-FeO—OH, $\beta$-FeO—OH, $\gamma$-FeO—OH und Eisenspinell $Fe_3O_4$.

Zu den besonders bevorzugten Cokatalysatoren gehören Eisen(II)-chlorid, Eisen(III)-chlorid, Eisenoxichlorid, sowie die Oxide und Oxidhydrate des dreiwertigen Eisens.

Im Falle der Verwendung der beispielhaft genannten, unter den Reaktionsbedingungen oft völlig inerten Oxide bzw. Hydroxide ist die Mitverwendung von aktivierenden Chloriden erforderlich. Es handelt sich hierbei um anionisch, als Chlorid gebundenes Chlor enthaltende Verbindungen, die unter den Reaktionsbedingungen mit den beispielhaft genannten Oxiden bzw. Hydroxiden unter deren zumindest teilweisen Überführung in die entsprechenden Chloride bzw. Oxichloride zu reagieren vermögen. Geeignete aktivierende Chloride sind beispielsweise Hydrochloride von tertiären Aminen des Molekulargewichtsbereichs 59 bis 300, Hydrochloride von primären Aminen, deren Substituenten den Substituenten der eingesetzten Harnstoffe vorzugsweise entsprechen — beispielsweise Anilinhydrochlorid bei Verwendung von Diphenylharnstoff-, Chlorwasserstoff, Eisen(II)-chlorid oder Eisen(II)-chlorid-Komplexe. Besonders gut geeignete aktivierende Chloride sind Pyridinhydrochlorid, Anilinhydrochlorid, das Hydrochlorid von 2,4-Diaminotoluol, Chlorwasserstoff, Eisen(II)-chlorid oder Eisen(II)-chlorid-Komplexe. Kombinationen der zuletzt genannten besonders bevorzugten aktivierenden Chloride mit den Oxiden und Oxidhydraten des dreiwertigen Eisen stellen besonders wertvolle Cokatalysatoren bb) dar.

Die Cokatalysatoren einschließlich der aktivierenden Chloride werden beim erfindungsgemäßen Verfahren im allgemeinen in Konzentrationen von 0,1 bis 20 Gew.-%, vorzugsweise 1 bis 5 Gew.-%, bezogen auf Reaktionsgemisch inklusive gegebenenfalls mitverwendeten Lösungsmittel eingesetzt. Der Anteil der aktivierenden Chloride liegt, falls diese überhaupt erforderlich sind, hierbei im

allgemeinen bei 0,05 bis 10, vorzugsweise 0,1 bis 2,5 Gew.-%.

Die erfindungsgemäße Umsetzung kann in Gegenwart oder in Abwesenheit eines Lösungsmittels durchgeführt werden. Im allgemeinen dient die vorzugsweise im Überschuß eingesetzte organische Hydroxylverbindung als Lösungsmittel. Die Mitverwendung inerter Lösungsmittel, die bis zu 80 Gew.-% des gesamten Reaktionsansatzes ausmachen können, ist jedoch auch möglich. Die Menge des Lösungsmittels muß, gleichgültig ob es sich um im Überschuß eingesetzte Hydroxylverbindung oder um inertes Lösungsmittel handelt, so bemessen sein, daß die Reaktionswärme der exothermen Urethanbildung ohne unzulässige Temperaturerhöhung abgeführt werden kann. Im allgemeinen wird daher das erfindungsgemäße Verfahren unter Verwendung einer Konzentration von Harnstoffverbindungen von 5 – 50 Gew.-%, vorzugsweise 5 – 20 Gew.-% und bei Einsatz von organischen Nitroverbindungen als Oxidationsmittel unter Verwendung einer Konzentration an Nitroverbindungen von 1 bis 20 Gew.-%, bevorzugt von 5 bis 10 Gew.-%, bezogen auf das Gesamtreaktionsgemisch inklusive dem Lösungsmittel durchgeführt.

Brauchbare Lösungsmittel sind gegen die Reaktionskomponenten und das Katalysatorsystem inerte Lösungsmittel, wie beispielsweise aromatische, cycloaliphatische und aliphatische Kohlenwasserstoffe, die gegebenenfalls durch Halogen substituiert sein können, wie Benzol, Toluol, Xylol, Chlorbenzol, Dichlorbenzol, Trichlorbenzol, Chlornaphthalin, Cyclohexan, Methylcyclohexan, Chlorcyclohexan, Methylenchlorid, Tetrachlorkohelnstoff, Tetrachloräthan, Trichlor-trifluoräthan u. a. Verbindungen.

Die Reaktionstemperatur liegt im allgemeinen zwischen 100° C und etwa 300° C, insbesondere zwischen 130° C und 250° C und besonders vorteilhaft im Bereich von 140° C bis 220° C. Der Druck muß so bemessen sein, daß stets das Vorliegen einer flüssigen Phase gewährleistet ist und liegt im allgemeinen im Bereich von 5 bis 500 bar, besonders vorteilhaft im Bereich von 30 bis 300 bar bei Reaktionstemperatur. Je nach eingesetztem primären Amin bzw. Hydroxyverbindung und gegebenenfalls organischer Nitroverbindung beträgt die für den quantitativen Umsatz benötigte Reaktionszeit zwischen wenigen Minuten und mehreren Stunden.

Die Umsetzung der Harnstoffe mit den Hydroxyverbindungen, Kohlenmonoxid und dem Oxidationsmittel zu Urethanen kann diskontinuierlich oder kontinuierlich durchgeführt werden.

Die diskontinuierliche Umsetzung kann im Hochdruckautoklaven mit geringen Mengen homogen gelöstem Edelmetall und einem ausreichenden Überschuß Katalysator, gegebenenfalls in Gegenwart eines aktivierenden Chlorids, durchgeführt werden. Im Reaktionsmedium unlösliche Verbindungen, wie beispielsweise Eisenoxide, bzw. Eisenoxidhydrate, können in Form eines feinen Pulvers zugegeben werden, die aktivierenden Zusatzstoffe in Form einer homogenen alkoholischen Lösung. Die Verteilung nicht gelöster überschüssiger Cokatalysatorbestandteile kann durch kräftiges Rühren oder durch Umpumpen des Reaktionsgemisches erfolgen. Die exotherme Reaktionswärme kann durch intern eingebaute Kühlaggregate oder im Falle des Umpumpens auch über einen externen Wärmeaustauscher abgeführt werden. Die Aufarbeitung und Katalysatorrückführung kann je nach Löslichkeit des erzeugten Urethans im Reaktionsgemisch auf verschiedene Weise erfolgen. Bei leichtlöslichen Urethanen kann beispielsweise nach beendeter Reaktion die Hauptmenge des bei tiefen Temperaturen schwerlöslichen Cokatalysatorgemisches samt dem größten Teil des adsorbierten Palladiums und des organischen Aminsalzes vom Reaktionsprodukt abgetrennt werden, beispielsweise durch Filtration oder Zentrifugieren, und in eine neue Umsetzung von primären Aminen Hydroxylverbindungen, Kohlenmonoxid und Oxidationsmittel wieder zurückgeführt werden. Das flüssige Reaktionsgemisch kann auf übliche Weise, z. B. durch fraktionierte Destillation, in Lösungsmittel, in die reinen Urethane und gegebenenfalls kleine Mengen von Nebenprodukten aufgetrennt werden, wobei diese Trennung diskontinuierlich oder kontinuierlich erfolgen kann. Im Destillationsrückstand sind kleine Mengen der im Reaktionsgemisch gelösten Cokatalysatorbestandteile und/oder Spuren von Edelmetall und/oder Edelmetallverbindungen enthalten, die wieder in die Umsetzung zurückgeführt werden können.

Im Falle von im Lösungsmittel bzw. überschüssiger Hydroxyverbindung schwer löslichen Urethanen kann die Aufarbeitung des Reaktionsgemisches in abgeänderter Weise erfolgen. Beispielsweise wird nach Entspannung unter Druck und höherer Temperatur, bei welchen die Urethane noch gelöst sind, jedoch das Katalysatorsystem Edelmetall/Cokatalysatorgemisch weitgehend ausfällt, die Hauptmenge Katalysator abfiltriert oder abzentrifugiert und dann durch Temperaturerniedrigung das schwerlösliche Urethan, gegebenenfalls zusammen mit geringen Mengen schwerlöslicher Nebenprodukte und restlichem Katalysator, auskristallisiert. Die Mutterlauge, die außer Lösungsmittel, bzw. der als Lösungsmittel abgewandten überschüssigen organischen Hydroxyverbindung, noch geringe Mengen Nebenprodukte, gelöste Urethan und gegebenenfalls gelöste Cokatalysatorbestandteile enthält, kann direkt oder nach vorheriger Entfernung leichtsiedender Nebenprodukte, beispielsweise durch Destillation, in die Oxycarbonylierung der primären Amine mit den Hydroxyverbindungen, Kohlenmonoxid und dem Oxidationsmittel zurückgeführt werden, wobei die dem vorherigen Umsatz entsprechende Menge primäres Amin, Hydroxyverbindung und gegebenenfalls Nitroverbindung als Oxidationsmittel, ergänzt wird. Höhersiedende Nebenprodukte, welche nicht durch Kristallisation entfernt werden, können durch destillative Aufarbeitung eines aliquoten Teiles der Mutterlauge als Destillationsrückstand kontinuierlich aus dem Rückführstrom entfernt werden.

Das ausgefallene Rohurethan kann beispielsweise durch Kristallisation aus einem das Urethan bei höheren Temperaturen lösenden, die Nebenprodukte und die katalysatorreste jedoch nicht lösenden Lösungsmittel wie beispielsweise Isooktan, Benzol, Toluol, Xylol, Chlorbenzol, Dichlorbenzol rekristallisiert werden. Die bei erhöhter Temperatur unlöslichen Rückstände können durch Oxidation in unlösliche Oxide, wie beispielsweise Eisenoxide, und ein aus den organischen Verunreinigungen resultierenden Abgas, das im wesentlichen aus Kohlendioxid, Sauerstoff, Stickstoff und gegebenenfalls leicht flüchtigen organischen Verunreinigungen besteht, umgewandelt werden. Das Abgas kann je nach Zusammensetzung direkt in die Atmosphäre abgelassen werden oder zusätzlich einer katalytischen Nachverbrennung zugeführt werden, in welcher restliche Verunreinigungen oxidativ entfernt werden. Die aus dem Rückstand erhaltene oxidische Verbindung, die gegebenenfalls noch geringe Mengen Edelmetall und/oder Edelmetallverbindung enthalten kann, wird wieder in die Oxycarbonylierung zurückgeführt.

Das bei der Oxycarbonylierung erhaltene Reaktionsgas, welches nicht umgesetztes Kohlenmonoxid, leichtsiedende organische Bestandteile, geringe Mengen Kohlendioxid und bei Verwendung von molekularem Sauerstoff als Oxidationsmittel auch geringe Mengen nichtumgesetzten Sauerstoff, sowie das zusätzlich mit eingebrachte Inertgas, wie beispielsweise Stickstoff, enthalten kann, wird beispielsweise nach Abtrennung der leichtsiedenden organischen Nebenprodukte und gegebenenfalls von Kohlendioxid, wieder auf Reaktionsdruck gebracht und in die Reaktion zurückgeführt unter Ergänzung der verbrauchten Anteile Kohlenmonoxid und gegebenenfalls molekularen Sauerstoff.

Die kontinuierliche Umsetzung kann in einer Kesselkaskade, einem Rohrbündelreaktor, mehreren hintereinander geschalteten Schlaufenreaktoren, in einem oder einer Serie hintereinander geschalteter adiabater Reaktionsrohren oder in einer Blasensäule durchgeführt werden. Die Wärmeabführung erfolgt beispielsweise entweder intern durch eingebaute Kühlaggregate, extern über einen Rohrbündelwärmetauscher oder adiabatisch über die Wärmekapazität des Reaktionsgemisches mit nachfolgender Abkühlung in externen Kühlaggregaten.

Die weitere Aufarbeitung kann wie oben beschrieben erfolgen, wobei sowohl eine kontinuierliche als auch eine diskontinuierliche Arbeitsweise angewandt werden kann.

Im Falle der bevorzugten Verwendung der erfindungsgemäßen Verfahrensprodukte als Zwischenprodukte zur Herstellung der entsprechenden Isocyanate ist ihre Reindarstellung oft überflüssig. Zur weiteren Verarbeitung kann es vielmehr ausreichend sein, die nach Abfiltrieren des Katalysators und gegebenenfalls Abdestillieren des Lösungsmittels anfallende Rohprodukte in die Weiterverarbeitung einzusetzen.

Das Verfahren wird durch die folgenden Beispiele illustriert, ohne damit die Erfindung auf die in den Beispielen gegebenen Bedingungen einzuschränken.

## Beispiele

### Beispiel 1

In einem 0,7-l-Autoklaven wurden 250 g eines Reaktionsgemisches folgender Zusammensetzung vorgelegt:

$2 \cdot 10^{-3}$ Gew.-% Palladiumchlorid, 3,7 Gew.-% Eisenoxidchlorid, 18,5 Gew.-% N,N'-Diphenylharnstoff und 77,8 Gew.-% Ethanol. Bei Raumtemperatur wurden 100 bar Kohlenmonoxid und 20 bar Luft aufgepreßt und 1 Stunde bei 180°C reagieren gelassen. Die gaschromatographische Analyse des flüssigen Reaktionsproduktes ergab einen Umsatz von 98% des eingesetzten Diphenylharnstoffs und eine Phenyl-urethan-Selektivität von 72%, bezogen auf Diphenylharnstoff, bzw. von 98%, bezogen auf umgesetztes Ethanol.

### Beispiel 2

Es wurde der gleiche Reaktionsansatz wie im Beispiel 1 verwendet, jedoch zweimal mit je 100 bar Kohlenmonoxid und 20 bar Luft je 1 Stunde umgesetzt, d. h. die gesamte Reaktionszeit bei 180°C betrug 2 Stunden. Man erhielt bei 99% Diphenylharnstoff-Umsatz Phenylurethan-Selektivitäten von 88%, bezogen auf Harnstoff, und von 98%, bezogen auf Ethanol.

### Beispiel 3

Es wurde wie in Beispiel 1 gearbeitet, jedoch als Kokatalysator ein Gemisch aus $\alpha$-$Fe_2O_3$ (2,4 Gew.-%) und Anilinhydrochlorid (3 Gew.-%) eingesetzt. Man erhielt bei quantitativem Diphenylharnstoff-Umsatz 80 Mol-% Phenylurethan, bezogen auf Diphenylharnstoff, bzw. 98 Mol-%, bezogen auf umgesetztes Ethanol.

## Beispiele 4 bis 8

In der folgenden Tabelle sind die Ergebnisse der Umsetzungen mit verschiedenen Kokatalysatoren auf der Basis Oxid und aktivierendes Chlorid enthalten. Es wurden jeweils 250 g eines Gemisches folgender Zusammensetzung in einem 0,7-l-Autoklaven eingesetzt:

$1{,}7 \cdot 10^{-3}$ Gew.-% $PdCl_2$, 2,4 Gew.-% zur Redoxreaktion befähigtes Oxid, 3 Gew.-% Anilinhydrochlorid und 13,5 Gew.-% N,N'-Diphenylharnstoff, 81,1 Gew.-% Ethanol. Es wurde bei jedem Versuch 3mal mit je 100 bar CO und 20 bar Luft (eingesetzt bei Raumtemperatur) je 1 Stunde bei 180°C umgesetzt (insgesamt je Versuch 3 Stunden Reaktionszeit bei 180°C). Man erhielt die in nachstehender Tabelle aufgeführten Ergebnisse.

| Beispiel Nr. | Kokatalysator-Oxid | Diphenylharnstoff-Umsatz % | Phenylurethan Selektivität Harnstoff | Ethanol |
|---|---|---|---|---|
| 4 | $V_2O_5$ | 99 | 93 | 84,4 |
| 5 | $MnO_2$ | 100 | 90 | 99,3 |
| 6 | $MoO_3$ | 100 | 85 | 77 |
| 7 | $WO_3$ | 99 | 82,4 | 94,1 |
| 8 | $Sb_2O_3$ | 99,5 | 94 | 87,4 |

## Beispiel 9

In einem 0,7-l-Edelstahlautoklaven wurden 250 g eines Reaktionsansatzes folgender zusammensetzung vorgelegt:

$2 \cdot 10^{-3}$ Gew.-% $PdCl_2$, 4 Gew.-% Eisenoxychlorid, 20 Gew.-% eines Gemisches von N,N'-Diphenylharnstoff und Nitrobenzol im Molverhältnis Diphenylharnstoff/Nitrobenzol = 2 : 1, sowie 76 Gew.-% Ethanol. Es wurden sodann bei Raumtemperatur 120 bar Kohlenmonoxid aufgepreßt und 1 Stunde bei 160°C umgesetzt. Die gaschromatographische Analyse zeigte, daß der Diphenylharnstoff quantitativ umgesetzt worden war und Phenylurethan in einer Selektivität von 96%, bezogen auf Diphenylharnstoff und Nitrobenzol, bzw. von 93%, bezogen auf Ethanol, gebildet worden war.

## Beispiele 10 bis 14

Die folgenden Beispiele verdeutlichen den Einfluß weiterer Edelmetalle auf die Oxicarbonylierung von N,N'-Diphenylharnstoff (DPH) mit Nitrobenzol (NB):

**Bedingungen:**

| | |
|---|---|
| **Kokatalysator:** | 3,7 Gew.-% Eisenoxychlorid |
| **Diphenylharnstoff:** | 14,93 Gew.-% |
| **Nitrobenzol:** | 4,33 Gew.-% |
| **Molverhältnis:** Diphenylharnstoff zu Nitrobenzol | 2 : 1 |
| **Alkohol:** | 77 Gew.-% Ethanol |
| **Kohlenmonoxid-Druck:** | 120 bar bei 20°C |
| **Einsatz Reaktionsgemisch:** | 270 g (im 0,7-l-Edelmetallautoklaven) |

Ergebnisse:

| Beispiel Nr. | Edelmetallverbindung (Gew.-%) | Tempe-ratur °C | Zeit h | Umsätze (%) DPH | NB | Phenylurethan-Selektivität (%) auf DPH + NB | Ethanol |
|---|---|---|---|---|---|---|---|
| 10 | $RhCl_3(2,7 \cdot 10^{-3})$ | 150 | 2 | 89 | 100 | 95 | 100 |
| 11 | $RhCl_3(2,7 \cdot 10^{-3})$ | 180 | 1 | 99 | 100 | 99 | 98 |
| 12 | $IrCl_3(3,7 \cdot 10^{-3})$ | 180 | 1 | 99 | 100 | 90 | 98 |
| 13 | $PtCl_4(3,7 \cdot 10^{-3})$ | 180 | 2 | 99 | 100 | 93 | 94 |
| 14 | $RuCl_3(3,7 \cdot 10^{-3})$ | 180 | 2 | 99 | 100 | 88 | 98 |

Beispiele 15 bis 22

**Die folgenden Beispiele verdeutlichen den Einfluß des Kokatalysators auf die Oxicarbonylierung von N,N'-Diphenylharnstoff (DPH) mit Nitrobenzol (NB).**

Bedingungen:

| | |
|---|---|
| Edelmetall: | $1,8 \cdot 10^{-3}$ Gew.-% $PdCl_2$ |
| DPH + NB: | 20 Gew.-% (in Ethanol) |
| Molverhältnis $\frac{DPH}{NB}$ | = 1,5 : 1 |
| Co-Druck: | 120 bar bei 20°C |
| Temperatur: | 180°C |
| Reaktionszeit: | 1 h |
| Einsatz-Gemisch: | 270 g (im 0,7-l-Edelstahlautoklaven) |

Ergebnisse:

| Beispiel Nr. | Kokatalysator (Gew.-%) | Umsätze (%) DPH | NB | Phenylurethan-Selektivität (%) auf DPH + NB | Ethanol |
|---|---|---|---|---|---|
| 15 | FeOCl (3,7) | 99 | 100 | 96 | 96 |
| 16 | $FeCl_3$ (3,6) | 99 | 100 | 90 | 90 |
| 17 | $FeCl_2 \cdot 4\,H_2O$ (5,5) | 99 | 100 | 82 | 97 |
| 18 | $VCl_3$ (1,9) | 99 | 100 | 92 | 94 |
| 19 | $CuCl_2$ (1,9) + $CeCl_3$ (1,5) | 98 | 37 | 70 | 97 |
| 20 | $\alpha$-$Fe_2O_3$ (2,5) + Anilin · HCl (3,3) | 99 | 90 | 98 | 99,5 |
| 21 | $V_2O_5$ (2,5) + Anilin · HCl (3,3) | 99 | 100 | 93 | 100 |
| 22 | $MoO_3$ (2,5) + Anilin · HCl (3,3) | 98 | 100 | 100 | 94 |

Beispiel 23

**In einem 0,7-l-Autoklaven wurden 250 g folgenden Gemisches vorgelegt: $2 \cdot 10^{-3}$ Gew.-% $PdCl_2$, 4 Gew.-% FeOCl, 14,8 Gew.-% eines Bisharnstoffes aus 2,4-Diaminotoluol und 2-Amino-4-nitrotoluol sowie 81 Gew.-% Ethanol. Die Nitrogruppen des eingesetzten Harnstoffes sind Oxidationsmittel für die Oxicarbonylierung der Harnstoff-Funktion. Es wurden 120 bar CO bei Raumtemperatur aufgepreßt und 2 Stunden bei 180°C reagieren gelassen. Der Bisharnstoff wird quantitativ umgesetzt. Es entsteht**

das Bisurethan des 2,4-Diisocyanotoluols in einer Selektivität von 50 Mol-% neben 10 Mol-% isomerer Aminourethane.

## Patentansprüche

1. Verfahren zur Herstellung von Urethanen durch Umsetzung von N,N'-disubstituierten Harnstoffen und mindestens eine Hydroxylgruppe enthaltenden organischen Verbindungen, dadurch gekennzeichnet, daß man diese Ausgangsverbindungen mit Kohlenmonoxid einer Oxicarbonylierung in Gegenwart von
a) molekularem Sauerstoff und/oder organischen Nitroverbindungen als Oxidationsmittel und
b) eines Katalysatorsystems welches
ba) aus einem Edelmetall und/oder einer Edelmetallverbindung der 8. Nebengruppe des Periodensystems der Elemente und
bb) einer unter den Reaktionsbedingungen zu Redoxreaktionen befähigten, von den Verbindungen ba) verschiedenen Verbindung von Elementen der 3. bis 5. Hauptgruppe und/oder der 1. bis 8. Nebengruppe des Periodensystems der Elemente besteht,
unterwirft.

2. Verfahren gemäß Anspruch 1, dadurch gekennzeichnet, daß man als N,N'-disubstituierte Harnstoffe symmetrische N,N'-Diarylharnstoffe und als Nitroverbindung eine aromatische Nitroverbindung verwendet, deren Arylrest den Arylresten des Harnstoffs entspricht.

3. Abänderung des Verfahrens gemäß Anspruch 1, dadurch gekennzeichnet, daß man anstelle der separaten Verwendung von N,N'-disubstituierten Harnstoffen und organischen Nitroverbindungen, Nitro-substituierte N,N'-Diarylharnstoffe verwendet.

4. Verfahren gemäß Anspruch 1 bis 3, dadurch gekennzeichnet, daß man als Katalysatorkomponente ba) Palladium, Rhodium, Palladiumverbindungen und/oder Rhodiumverbindungen verwendet.

5. Verfahren gemäß Anspruch 1 bis 4, dadurch gekennzeichnet, daß man als Katalysatorkomponente bb) Oxichloride von unter den Reaktionsbedingungen zu Redoxreaktionen befähigten Elementen der 3. bis 5. Hauptgruppe oder 1. bis 8. Nebengruppe des Periodensystem verwendet.

6. Verfahren gemäß Anspruch 1 bis 4, dadurch gekennzeichnet, daß man als Katalysatorenkomponente bb) (i) Oxide und/oder Hydroxide von unter den Reaktionsbedingungen zu Redoxreaktionen befähigten Elementen der 3. bis 5. Hauptgruppe oder 1. bis 8. Nebengruppe des Periodensystem in Kombination mit (ii) anionisch, als Chlorid gebundenes Chlor enthaltenden Verbindungen, die unter den Reaktionsbedingungen zur Aktivierung der Oxide bzw. Hydroxide unter Chloridbildung geeignet sind, verwendet.

7. Verfahren gemäß Anspruch 1 bis 5, dadurch gekennzeichnet, daß man als Katalysatorenkomponente bb) Eisenoxychlorid verwendet.

8. Verfahren gemäß Anspruch 1 bis 7, dadurch gekennzeichnet, daß man als Hydroxylgruppen aufweisende organische Verbindungen einen einwertigen, primären, aliphatischen Alkohol mit 1 bis 6 Kohlenstoffatomen verwendet.

9. Verfahren gemäß Anspruch 1 bis 8, dadurch gekennzeichnet, daß man die Umsetzung im Temperaturbereich zwischen 100 und 300° C und bei einem Druck zwischen 5 und 500 bar durchführt.

## Claims

1. Process for the preparation of urethanes by the reaction of N,N'-disubstituted ureas and organic compounds containing at least one hydroxyl group, characterised in that these starting compounds are subjected to oxycarbonylation with carbon monoxide in the presence of
a) molecular oxygen and/or organic nitro compounds as oxidizing agents and
b) a catalyst system consisting of
ba) a noble metal and/or a noble metal compound of the 8th subgroup of the periodic system of elements and
bb) a compound of elements of the 3rd to 5th main group and/or 1st to 8th subgroup of the periodic system of elements which compound is capable of undergoing Redox reactions under the reaction conditions and which is different from the compounds ba).

2. Process according to claim 1, characterised in that the N,N'-disubstituted ureas are symmetric N,N'-diaryl ureas and the nitro compound used is an aromatic nitro compound having the same aryl group as the urea.

3. Modification of the process according to Claim 1, characterised in that instead of using separate N,N'-disubstituted ureas and organic nitro compounds, nitro substituted N,N'-diaryl ureas are used.

4. Process according to Claims 1 to 3, characterised in that the substances used as catalyst component ba) are palladium rhodium, palladium compounds and/or rhodium compounds.

5. Process according to Claims 1 to 4, characterised in that the substances used as catalyst component bb) are oxychlorides of elements of the 3rd to 5th main group or 1st to 8th subgroup of the

periodic system, which elements are capable of Redox reactions under the reaction conditions.

6. Process according to Claims 1 to 4, characterised in that the substances used as catalyst component bb) are: (i) oxides and/or hydroxides of elements of the 3rd to 5th main group or 1st to 8th subgroup of the periodic system which elements are capable of Redox reactions under the reaction conditions, in combination with (ii) compounds containing chlorine bound anionically as chloride, which compounds are capable, under the reaction conditions, of activating the oxides or hydroxides with formation of chloride.

7. Process according to claims 1 to 5, characterised in that iron oxychloride is used as catalyst component bb).

8) Process according to claims 1 to 7, characterised in that the substances used as organic compounds having hydroxyl groups are monohydric primary, aliphatic alcohols having 1 to 6 carbon atoms.

9. Process according to claims 1 to 8, characterised in that the reaction is carried out in the temperature range of from 100 to 300° C and at a pressure of from 5 to 500 bar.

**Revendications**

1. Procédé pour préparer des uréthannes par réaction d'urées N,N'-disubstituées et de composés organiques protant au moins un groupe hydroxy, caractérisé en ce que l'on soumet ces composés de départ avec l'oxyde de carbone à une oxycarbonylation en présence de:

a) de l'oxygène moléculaire et/ou des composés organiques nitrés servant d'agents oxydants et
b) un système catalyseur consistant en
ba) un métal noble et/ou un composé de métal noble du 8e sous-groupe de la Classification Périodique des Eléments et
bb) un composé différemt des composés ba), apte à des réactions d'oxydo-réduction dans les conditions de la réaction, des éléments du 3e au 5e groupe principal et/ou du ler au 8e sous-groupe de la Classification Périodique des Eléments.

2. Procédé selon la revendication 1, caractérisé en ce que l'on utilise en tant qu'urées N,N'-disubstituées des N,N'-diarylurées symétriques et en tant que composé nitré un composé aromatique nitré dont le reste aryle correspond aux reste aryle de l'urée.

3. Variante du procédé selon la revendication 1, caractérisée en ce que, au lieu d'utiliser séparément des urées N,N'-disubstituées et des composés organiques nitrés, on utilise des N,N'-diarylurées portant des substituants nitro.

4. Procédé selon les revendications 1 à 3, caractérisé en ce que l'on utilise en tant que composant ba) du catalyseur le palladium, le rhodium, des composés du palladium et/ou des composés du rhodium.

5. Procédé selon les revendications 1 à 4, caractérisé en ce que l'on utilise en tant que composant bb) du catalyseur des oxychlorures des éléments du 3e au 5e groupe principal ou du ler au 8e sous-groupe de la Classification Périodique, aptes à des réactions d'oxydo-réduction dans les conditions de la réaction.

6. Procédé selon les revendications 1 à 4, caractérisé en ce que l'on utilise en tant que composant bb) du catalyseur (i) des oxydes et/ou hydroxydes des éléments du 3e au 5e groupe principal ou du ler au 8e sous-groupe de la Classification Périodique, aptes à des réactions d'oxydo-réduction dans les conditions de la réaction, en combinaison avec (ii) des composés anioniques, contenant du chlore combiné à l'état de chlorure, approprié à l'activation des oxydes ou hydroxydes avec formation de chlorures dans les conditions de la réaction.

7. Procédé selon les revendications 1 à 5, caractérisé en ce que l'on utilise en tant que composant bb) du catalyseur l'oxychlorure de fer.

8. Procédé selon les revendications 1 à 7, caractérisé en ce que l'on utilise en tant que composé organique portant des groupes hydroxy un alcool aliphatique primaire monovalent contenant de 1 à 6 atomes de carbone.

9. Procédé selon les revendications 1 à 8, caractérisé en ce que l'on effectue la réaction dans un intervalle de température de 100 à 300° C et sous une pression de 5 à 500 bars.